# EUROPEAN PATENT APPLICATION

(11) **EP 4 361 282 A1**
(43) Date of publication of application: **01.05.2024**
(21) Application number: 22203669.1
(22) Date of filing: 25.10.2022
(51) Int. Cl.: C12Q 1/682

(54) **INCREASING THE SENSITIVITY OF A CRISPR-CAS NUCLEAIC ACID DETECTION SYSTEM**

(71) Applicant: Nederlandse Organisatie voor toegepast-natuurwetenschappelijk Onderzoek TNO, 2595 DA 's-Gravenhage (NL)
(72) Inventor: VAN LEEUWEN, Hans Christiaan, 2595 DA `s-Gravenhage (NL)
(74) Representative: V.O.

(57) **Abstract**

The disclosure provides an amplification system for a nucleic acid detection system. This amplification system is particularly useful to increase the sensitivity of the nucleic acid detection system. Furthermore, the disclosure provides a method for detecting nucleic acids.

## Description

### FIELD OF THE INVENTION

The disclosure provides an amplification system for a CRISPR-Cas nucleic acid detection system. This amplification system is particularly useful to increase the sensitivity of the nucleic acid detection system.

Furthermore, the disclosure provides a method for detecting one or more nucleic acids of interest in a sample, wherein the method comprises a CRISPR-Cas system and a signal amplification system.

### BACKGROUND OF THE INVENTION

Clustered Regularly Interspaced Short Palindromic Repeats (CRISPR) and CRISPR-associated (CRISPR-Cas) nucleases constitute a class of adaptive immune systems used by bacteria and archaea. A number of CRISPR-Cas systems have been adapted to, for example, genome engineering, serving as programmable nucleases cleaving desired genomic sequences or as specific DNA binders enabling transcriptional regulation, base editing, or imaging.

In addition, CRISPR-Cas systems can be used in detecting a myriad of pathogens, such as viral and bacterial pathogens, by harnessing cleaved nucleic acid fragments for use in diagnostic testing.

CRISPR-Cas enzymes have been used extensively for the development of fast point-of-care diagnostic nucleic acid detection tests. CRISPR-Cas enzymes, in conjunction with a targeting guide RNA, recognize specific nucleic acid sequences and subsequently activate inbuilt specific and non-specific nucleic acid cleavage. The non-specific nucleic acid cleavage-activity of CRISPR-Cas enzymes can be utilised to generate a measurable, visible signal. A commonly applied read-out of CRISPR-Cas-tests can be achieved using Förster resonance energy transfer (FRET) technique as a reporter method. With FRET, DNA-oligonucleotides are labelled with a fluorophore at one end and a quencher at the other end. After CRISPR-Cas-activation and subsequent cleavage, the reporter-oligonucleotide starts to fluorescence, which can be measured in spectrofluorometers.

Several diagnostic CRISPR-Cas assays exist, such as DETECTR (DNA Endonuclease Targeted CRISPR Trans Reporter), SHERLOCK (Specific High Sensitive Enzymatic Reporter) and HOLMES (one-HOur Low-cost Multipurpose highly Efficient System). These assays rely on synthetic RNA/DNA oligonucleotides that have a reporter fluorophore (donor) on one end and a quencher (acceptor) in proximity on the other end.

While the pathogen nucleic acid lower detection-limit of Cas12 diagnostics is reported in the nanomolar to the picomolar range, the sensitivity of these tests can be increased by target pre-amplification strategies, reaching femtomolar to even attomolar sensitivity. Target pre-amplification (PCR, LAMP, RPA) requires designing and testing target-specific primer-pairs on top of designing a CRISPR-Cas guide RNA for the desired target sequence. These extra steps reduce the simplicity to change the target of CRISPR-Cas diagnostic tests. Moreover, pre-amplification adds steps in the protocol of CRISPR-Cas-diagnostic tests requiring extra waiting time, equipment and increased complexity.

For example, Notomi *et al.* (2000) describes a method to increase the sensitivity of diagnostics test by target pre-amplification strategies. This method describes loop-mediated isothermal amplification (LAMP) of DNA. The method amplifies DNA under isothermal conditions and employs a DNA polymerase and a set of four specially designed primers that recognize a total of six distinct sequences on the target DNA. With LAMP cycling the target DNA is copied and the final products are stem-loop DNAs.

A further exemplary method of isothermal amplification is described in Gootenberg *et al.* 2017. Gootenberg *et al.* combines the collateral effect of Cas13a with isothermal amplification to establish a CRISPR-based diagnostic (CRISPR-Dx), providing rapid DNA or RNA detection with attomolar sensitivity and single-base mismatch specificity.

A further exemplary method is described by Shi *et al.* 2021, describing a CRISPR-Cas-powered catalytic nucleic acid circuit, namely, CRISPR-Cas-only amplification network (CONAN), for isothermally amplified detection of genomic DNA. This method has a positive feedback circuit with exponential dynamic in CONAN.

The exemplary methods make use of amplification strategies requiring extra waiting time, equipment and increased complexity.

Hence, there is a need for a system or method to increase the sensitivity of the diagnostics tests, but omitting extra pre-amplification and waiting steps.

### SUMMARY OF THE INVENTION

The disclosure provides the following preferred embodiments. However, the invention is not limited to these embodiments.

In some embodiments the disclosure provides a nucleic acid detection system, comprising a clustered regularly interspaced short palindromic repeat (CRISPR)- CRISPR-associated nuclease (Cas) system (CRISPR-Cas system), and a signal amplification system, wherein:
- the CRISPR-Cas system comprises an effector protein and a target-specific guide RNA (gRNA) comprising a guide sequence capable of targeting the effector protein to a target sequence, and the effector protein exhibiting target-activated collateral nucleic acid cleavage activity;
- the signal amplification system comprises a partial CRISPR RNA (crRNA) recognition site-oligo (POISER-oligo, Partial Or Incomplete Sites for crRNA recognition) and a POISER-oligo-specific gRNA comprising a guide sequence capable of targeting an effector protein to the POISER-oligo further comprising a polynucleotide tail, and the effector protein exhibiting target-activated collateral nucleic acid cleavage activity; and a template-independent polymerase.

In some embodiments the disclosure provides a signal amplification system for a nucleic acid detection system, comprising
- a partial crRNA recognition site-oligo (POISER-oligo),
- a POISER-oligo-specific guide RNA (gRNA) comprising a guide sequence capable of targeting an effector protein to the POISER-oligo further comprising a polynucleotide tail; and a non-template directed polymerase.

In some embodiments the disclosure provides the nucleic acid detection system as described herein or signal amplification system as described herein, wherein the POISER-oligo comprises 9-15 nucleotides at least partially complementary to the POISER-oligo-specific guide RNA.

In some embodiments the disclosure provides the nucleic acid detection system or signal amplification system as described herein, wherein the POISER-oligo is blocked at the 3'-terminus, *e*.*g*. by phosphorylation, circularisation, RNA nucleotides, 3'ddC, 3' Inverted dT, 3'C3spacer, 3'amino, and 3' phosphorylation.

In some embodiments the disclosure provides the nucleic acid detection system or signal amplification system as described herein, wherein the POISER-oligo is single stranded DNA, single stranded RNA, or single stranded hybrid DNA-RNA, preferably single-stranded DNA.

In some embodiments the disclosure provides the nucleic acid detection system or signal amplification system as described herein, wherein the POISER-oligo is at least partially protected with a backbone modification from degradation, preferably with a phosphorothioate backbone modification.

Preferably, the nucleic acid detection system or signal amplification system, further comprising a reporter molecule.

In some embodiments the disclosure provides the nucleic acid detection system or signal amplification system as described herein, wherein the POISER-oligo is a nucleic acid fluorescence detection system or a FRET detection system, preferably wherein the POISER-oligo is blocked at the 3'-terminus with a fluorophore or quencher of a FRET reporter assay.

In some embodiments the disclosure provides the nucleic acid detection system as described herein, wherein the CRISPR-Cas system comprises a Cas enzyme with collateral cleavage activity as effector protein, preferably wherein the effector protein is a class 2 type V or type VI Cas enzyme, preferably Cas12, Cas 13, and/or Cas14, more preferably wherein the effector protein is Cas12a.

In some embodiments the disclosure provides the nucleic acid detection system or signal amplification system as described herein, wherein the effector protein of the CRISPR-Cas system and the effector protein of the signal amplification system are identical.

In some embodiments the disclosure provides the nucleic acid detection system or signal amplification system as described herein, wherein the template independent polymerase comprises a non-template directed DNA polymerase and/or a non-template directed RNA polymerase, more preferably terminal deoxynucleotidyl transferase (TdT) and/or Pοlθ terminal transferase or a derivative thereof, preferably the nucleic acid detection system or signal amplification system further comprises nucleotides.

In some embodiments the disclosure provides the nucleic acid detection system as described herein, wherein the target sequence comprises nucleic acids, preferably DNA and/or RNA, preferably single stranded or double stranded DNA.

In some embodiments the disclosure provides the nucleic acid detection system as described herein, wherein the target sequence comprises nucleic acids of a pathogen, oncogene and/or genetic pathology, preferably a nucleic acid of a pathogen.

In some embodiments the disclosure provides the use of the nucleic acid detection system as described herein as a diagnostic test.

In some embodiments the disclosure provides a method for detecting one or more nucleic acids of interest in a sample, wherein the method comprises a CRISPR-Cas system and a signal amplification system, the method comprising the following steps:
i) adding to a sample the CRISPR-Cas system, signal amplification system, template-independent-polymerase and a reporter molecule according to any one of the preceding claims,
ii) read-out of the reporter.

In preferred embodiments the nucleic acids of interest are obtained from a biological sample or from an environmental sample.

### BRIEF DESCRIPTION OF THE DRAWINGS

**Figure 1****:** Examples of various POISER-oligo's. POISER oligonucleotides form an initiation point for TdT nucleotide elongation after cleavage by a Cas enzyme. The newly formed hybrid product is recognised by a second specific CRISPR-Cas crRNA.
**Figure 2****:** Schematic representation of the Feedback-loop to boost CRISPR-Cas cleavage activity thereby increasing pathogen detection sensitivity. TdT = terminal deoxynucleotidyl transferase; F = fluorophore, Q = quencher; OH = hydroxyl; T = deoxythymidine triphosphate; C = deoxycytidine triphosphate; ↓ = Cas collateral cleavage; * = blocked 3'OH end. POISER = Partial Or Incomplete Sites for crRNA recognition.
**Figure 3****:** Illustration of an optional 4th step in the feedback loop described in Fig. 2.
**Figure 4****:** A FRET fluorescent produced by Cas12a trans-cleavage activity is influenced by the number of phosphorothioate backbone interactions in the crRNA-target oligonucleotide.
**Figure 5****:** Cas12a unblocks ssDNA 3'OH end only in presence of 2 phosphodiester(PD)-nucleotides (NTs) at the 3' end. The ability of Cas12a to cleave one or two PD-nucleotides at the 3' end of a PS-oligo (yellow) is compared. The oligos contain a 5' end FAM (F) label that allows visualisation on an LFA strip. The signal is compared for reactions where Cas12a is activated using an ALF crRNA directed at the ALF gene (anthrax lethal factor). Cas12a is activated (+ pALF) and remained inactive (-pALF). NT = nucleotide; pALF = plasmid containing Anthrax lethal factor gene.
**Figure 6****:** Adding POISER2b to CRISPR-Cas12a boosts signal generated in pathogen detection test.

### DETAILED DESCRIPTION OF THE DISCLOSED EMBODIMENTS

The present disclosure provides a CRISPR-Cas nucleic acid detection system which has a high sensitivity to detect a target sequence of a target. In particular, the nucleic acid detection system has a low detection limit for a nucleic acid target, in particular nucleic acids of pathogens, oncogenes and/or genetic pathologies.

Furthermore, it is an objection of the invention to provide a method for detecting nucleic acids of a target with a high sensitivity, but omitting extra pre-amplification and waiting steps.

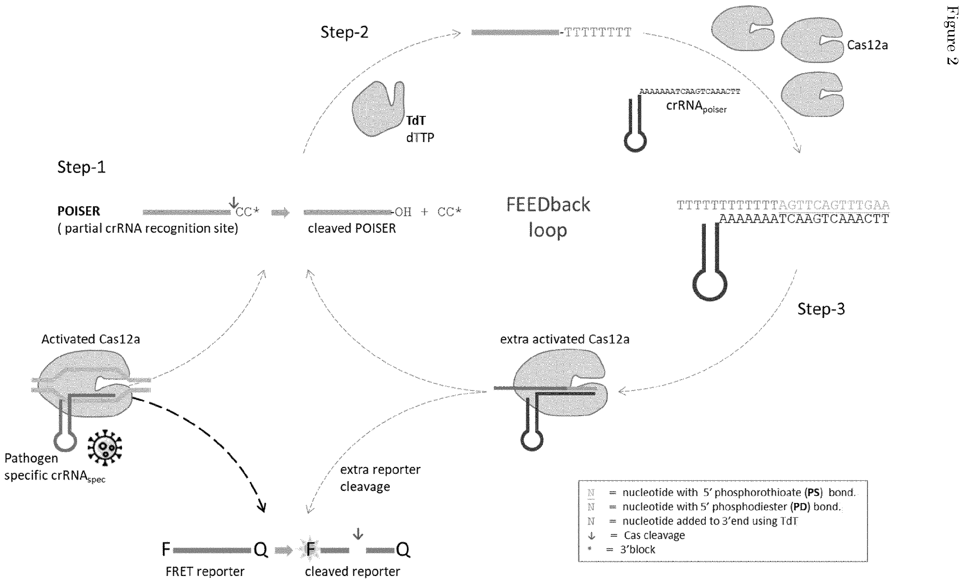

In an exemplary embodiment the POISER dependent signal amplification revolves around generating a positive feedback-loop after target recognition by the pathogen CRISPR-Cas-crRNA complex. This feedback loop comprises of three components as shown in Fig. 2. This embodiment supports the disclosure, but does not limit the scope of the invention.

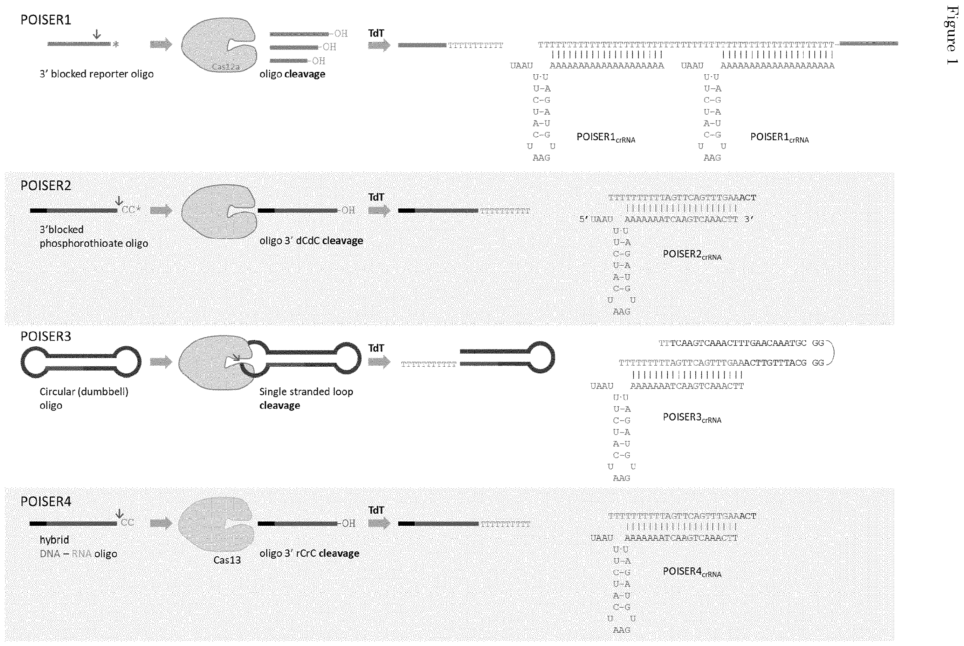

In this exemplary embodiment the first component (component 1) of the feedback-loop comprises a synthetic (complete or partial single-stranded) DNA/RNA POISER-oligo with its 3'-end blocked. The POISER oligonucleotide sequence of POISER2 (Fig. 1 and 2), comprises of 13 nucleotides of either G, C, A or T nucleotides. The POISER sequence order is irrelevant as long as the sequence is complementary to the POISERcrRNA (see component 3). POISER oligonucleotides can be as long 15 nucleotides. Longer POISER oligonucleotides will (auto-) activate Cas-POISERcrRNA complexes without the occurrence of pathogen recognition. The POISER-oligo used is single-stranded DNA but single-stranded-RNA or hybrid DNA-RNA might be used.

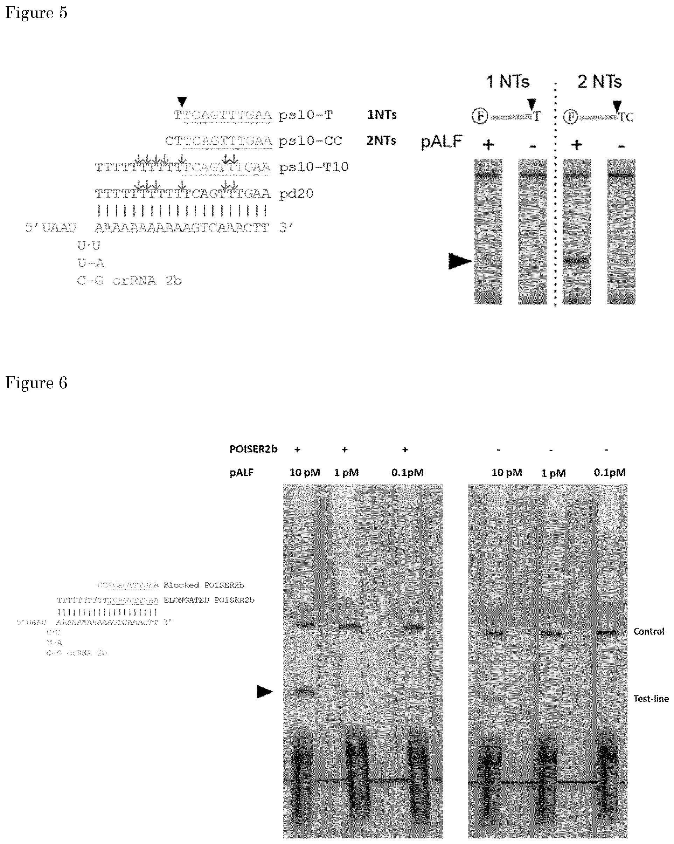

To prevent non-specific immediate TdT elongation, the POISER is initially 'blocked' at its 3' end (*i*.*e*., missing a functional 3'OH). Several methods for oligo blocking as known by people skilled in the art include: 3'ddNTP, 3' Inverted dT, 3'C3spacer, 3'Amino, and 3' phosphorylation. Exemplary POISER-oligo's as shown in Figs. 5 and 6 use synthetic 3'poshorylation plus enzymatic addition of ddCTP [ddNTPs] plus TdT for complete block.

To ensure the conservation of the POISER crRNA-complementary sequence (in case of POISER2 and POISER4, Fig. 1), part of the POISER2-oligo can be protected with a phosphorothioate (PT) backbone (see preliminary results). One or more nucleotides at the 3'-end need a standard phosphodiester bond in order to be cleaved by the activated Cas enzymes. Preferred is 2 nucleotides.

The second component (component 2) of the feedback-loop comprises the enzyme terminal deoxynucleotidyl transferase (TdT). This template independent polymerases is essential to add new nucleotides to the 3' POISER end.

The third component (component 3) of the feedback-loop comprises Cas loaded with a POISER-poly-nucleotide (poly-T/polyC/ polyG/ polyA) specific crRNA, POISERcrRNA.

Any Cas enzyme (Cas12x, Cas13, Cas14) with collateral cleavage activity can be used. Rational is that the same Cas enzyme is used as for the pathogen recognition.

The POISERcrRNA is complementary to the POISER-oligo as well as the newly TdT-added nucleotides and contains a specific stem-loop sequence, essential for Cas enzyme recognition as known to people skilled in the art.

In an exemplary embodiment the feed-back-loop consists of three steps which start after Cas-crRNA dependent pathogen recognition and collateral cleavage activation (Fig. 2). The steps in the feedback loop comprise:
Step 1 comprises the POISER-oligo (component 1), with a 3'blocked end, is cleaved by the collateral cleavage activity of the CRISPR-Cas enzyme activated by the presence of the pathogen. After cleavage and removal of the 3'block, TdT can initiate synthesis of poly-T tails (or G-tail, C-tails, A-tails), completing the POISER target recognition site.
Step 2 comprises TdT (component 2), a template-independent DNA polymerase, that can elongate free 3'OH (hydroxyl) ends of single-stranded DNA-oligo's by incorporating any deoxynucleotide triphosphate (dNTPs).

By adding dNTPs (in this case dTTP), the POISER "half-"site is elongated with T-nucleotides thereby creating a new site. This newly formed chimeric half-site with a T-tail is now recognised by the POISER crRNA, base-pairing with both the half-site and the newly added poly-T sequence (or poly-G, poly-C, poly-A).

Step 3 comprises final step in the positive feedback loop is the POISER-poly-T recognition by the POISERcrRNA-Cas complexes (component 3). POISER crRNAs are designed complementary to the elongated POISER-poly-T 'hybrid', meaning that the Cas enzyme loaded with POISER-crRNA will become activated, increasing the cleavage activity of Cas-complexes present. More activated Cas proteins mean that more FRET reporter will be cleaved, amplifying the signal and lowering the detection limit. Any reporter assay depending on Cas collateral cleavage assays will be enhanced.

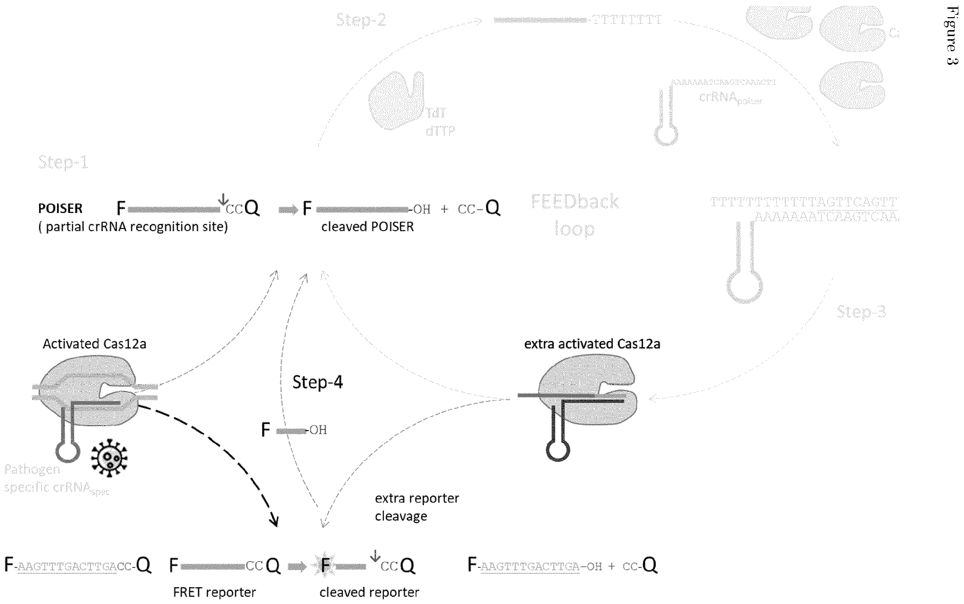

The exemplary embodiment may comprise an optional step 4 comprising addition of a 3'block can also be effectively achieved using a fluorophore or quencher identical to those used in the FRET reporter. Therefore it seems credible that the FRET reporter itself can be constructed as a POISER-oligo. Thus after cleavage of this FRET reporter, the 5'part can function as a cleaved POISER from which TdT starts polymerisation of a T-tail (Fig. 3). Thus the reporter molecule itself can also function as the POISER-oligo.

The disclosure provides a nucleic acid detection system comprising a CRISPR-Cas system and a signal-amplification system. Furthermore, the disclosure provides a signal amplification system. For example, the signal amplification system can be used in a CRISPR-Cas nucleic acid detection system.

In particular, the disclosure provides a nucleic acid detection system, comprising a clustered regularly interspaced short palindromic repeat (CRISPR)- CRISPR-associated nuclease (Cas) system (CRISPR-Cas system), and a signal amplification system, wherein: the CRISPR-Cas system comprises an effector protein and a target-specific guide RNA (gRNA) comprising a guide sequence capable of targeting the effector protein to a target sequence, and the effector protein exhibiting target-activated collateral nucleic acid cleavage activity (capable of cleaving nucleic acid molecules to generate nucleic acid fragments); wherein the signal amplification system comprises a partial CRISPR RNA (crRNA) recognition site-oligo (POISER-oligo) and a POISER-oligo-specific gRNA comprising a guide sequence capable of targeting an effector protein to the POISER-oligo further comprising a polynucleotide tail, and the effector protein exhibiting target-activated collateral nucleic acid cleavage activity; and a template-independent polymerase.

In some embodiments the disclosure provides a signal amplification system in a nucleic acid detection system, comprising a partial crRNA recognition site-oligo (POISER-oligo), a POISER-oligo-specific guide RNA having a guide sequence being capable of targeting an effector protein to an POISER-oligo with a polynucleotide tail; and a template-independent polymerase exhibiting catalytic activity capable of transferring nucleotide to the fragments to form polynucleotide tails.

The term "nucleic acid detection system" as used herein refers to a system for detecting a target nucleic acid. The target nucleic acid is recognised by the detection system resulting in a signal, which can be detected. The nucleic acid detection system of the invention comprises a CRISPR Cas-system and a signal amplification system.

The CRISPR-Cas system comprises an effector protein. The term "effector" protein as used herein refers to a protein that has cleavage activity. In particular, the effector protein can cleave nucleic acid molecules, such as DNA and RNA molecules. A typical example of an effector protein is a Cas enzyme.

In general, a CRISPR-Cas system is characterised by components that promote the formation of a so-called CRISPR complex, triggered by a target sequence. Typically, a CRISPR complex comprises a complex of an effector protein and a guide RNA. As used herein, the CRISPR-Cas system comprises an effector protein and one or more guide RNAs that are capable of guiding the effector protein to bind to a target molecule, such as a nucleic acid. The guide RNA has a guide sequence which is capable of targeting an effector protein to a target sequence. The target sequence is part of a target, *e.g.* a pathogen, an oncogene and/or genetic pathology. Typically, a guide-target hybrid is formed. In particular, the CRISPR-Cas system comprises a guide RNA having a guide sequence which is capable of targeting an effector protein to a target sequence, wherein preferably the target sequence is a nucleotide sequence of a target molecule, such as a nucleotide sequence of a nucleic acid, *e.g.,* a pathogenic nucleic acid, a genetic nucleic acid coding for an oncogene or genetic pathology.

CRISPR-Cas systems are commonly categorised in two classes (1 and 2). The categorisation of CRISPR-Cas systems is based on, for example, the constitution of effector modules of single, large, multidomain proteins that are generally derived from genetic elements. Class 1 CRISPR-Cas systems contain a multi subunit Cas nuclease, or effector protein. Class 2 CRISPR-Cas systems are characterised by a single subunit effector protein. Exemplary effector proteins that belong to class 2 CRISPR-Cas systems are type II Cas9 and Cas9-like proteins, type V Cas12 and Cas12-like proteins, such as subtype V-A Cas12 (Cpf1, or Cas12a), subtype V-B Cas12 (Cas12b, or C2c1) and subtype V-C Cas12 (C2c3), subtype V-F Cas12 (Cas12f, or Cas14 or C2c10) and type VI Cas13 and Cas13-like proteins, such as Cas 13a (C2c2) and Cas13b (C2c6).

In particular, the detection system as described herein comprises a class 2 CRISPR-Cas system. Preferably, the CRISPR-Cas system is a type V or VI system. More preferably, the CRISPR-Cas system is a class 2 type V system. In case of a class 2 system, the effector protein may particularly be Cas12 or an effector protein having similar cleavage activity as Cas12. The Cas12 or Cas12-like effector protein may, for example, be selected from Cas12a, Cas12b, Cas12c, Cas12d Cas12e, and Cas12f. Preferably, the CRISPR-Cas system comprises a C12a effector protein or an effector protein having similar cleavage activity as Cas12a.

The nucleic acid detection system as described herein may comprise more than one CRISPR-Cas system, for example, wherein the more than one CRISPR-Cas systems are different. Preferably, the detection system comprises one CRISPR-Cas system.

The programmability and specificity of the RNA guided class 2 Cas effector proteins, such as Cas12a, make them suitable switchable nucleases for specific cleavage of nucleic acids. The class 2 Cas effector proteins, such as Cas12a, may be engineered to provide and take advantage of improved collateral non-specific cleavage of DNA, preferably ssDNA. Accordingly, engineered class 2 Cas effector proteins, such as Cas12a, may provide suitable platforms for nucleic acid detection.

In some embodiments the effector protein of the CRISPR-Cas system and the effector protein of the signal amplification system are identical. The CRISPR-Cas system and the signal amplification system can use the same effector protein. For example a Cas enzyme can be used for target (e.g. pathogen) recognition and for the signal amplification system.

In some embodiments the effector protein of the CRISPR-Cas system and the effector protein of the signal amplification system are different effector proteins. The CRISPR-Cas system and the signal amplification system can use multiple effector proteins, preferably two effector proteins. For example the Cas enzyme used for target (*e*.*g*. pathogen) recognition is a different Cas enzyme than the Cas enzyme used for the signal amplification system. In some embodiments it may be possible to use a set of effector proteins, for example a set of Cas enzymes.

The term "guide sequence" as used herein is meant to refer to a nucleotide sequence of a guide RNA molecule. The guide sequence comprises a sequence that is at least partially complementary to the sequence of the target molecule. The guide sequence is therefore considered to be the key to bind an effector protein, comprising the guide RNA, to a target molecule, *e*.*g*., a nucleic acid, through sequence pairing.

A guide RNA (also referred herein as gRNA) as used herein is characterised by comprising any nucleotide sequence, or guide sequence, having sufficient complementarity with the nucleotide sequence of a target molecule to hybridise and/or pair, and to direct sequence specific binding of a molecule-targeting effector protein to the target molecule. The guide sequence may be a full-length guide sequence or a truncated guide sequence.

The degree of complementarity of the guide sequence to a given target sequence, when optimally aligned using a suitable alignment algorithm, may be 50 % or more, such as 60 % or more, 75 % or more, 80 % or more, 85 % or more, 90 % or more, 95 % or more, 97.5 % or more, or 99 % or more. In particular, the degree of complementarity may be 75 % or more, such as 85-100 %, 90-100 %, or 95-100 %. Preferably, the degree of complementarity is 97.5 % or more, 98 % or more, 98.5 % or more, 99 % or more, 99.5 % or more, or 99.7 % or more. More preferably, the degree of complementarity is about 100 %. Optimal alignment may be determined with the use of any suitable algorithm for aligning sequences, non-limiting example of which include the Smith Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net).

The CRISPR-Cas system in the detection system as described herein may comprise one or more guide RNAs which are capable of forming a wobble base pair with a target sequence. That is, such guide RNAs do not completely follow Watson-Crick base pairing. The CRISPR-Cas system may comprise one or more gRNAs that do follow Watson-Crick base pairing and one or more gRNAs that do not.

The total length of guide RNA may be at least 10 nucleotides and may be 100 nucleotides or less, such as 15-85 nucleotides. In particular, the guide RNA may be at least 15 nucleotides, such as 20 70, such as 30-65, or 40-50. The length of guide RNA can be 35 or more and 45 or less. Preferably, the guide RNA is between 15 and 30 nucleotides, such as 15-25 nucleotides.

The length of the guide sequence of the guide RNA may be at least 10 nucleotides and may be 100 nucleotides or less, such as 15-50 nucleotides. In particular, the guide sequence is at least 15 nucleotides, such as 16-35, such as 17-30. Preferably, the guide sequence is 18 or more and 25 or less.

The guide RNA may comprise naturally and/or non-naturally occurring nucleotides, nucleotide analogues, and/or chemical modifications. The non-naturally occurring nucleotides, nucleotide analogues, and/or chemical modifications may be located outside the guide sequence. Non-naturally occurring nucleotides and/or nucleotide analogues may be modified at the ribose, any or all of the phosphate groups, and/or nitrogenous base.

In the present disclosure the terms "guideRNA (gRNA)" and "CRISPR RNA (crRNA)" are used. The gRNA consists of two types of RNA, the crRNA and trans-activating CRISPR RNA (tracrRNA). The crRNA is complementary to the target DNA sequence and tracrRNA serves as a binding site of the Cas enzyme.

In some embodiments the CRISPR-Cas system of the invention comprises a target-specific guide RNA. The target-specific guide RNA can target a nucleic acid sequence of a target. The target-specific guide RNA may comprise one or more gRNAs. In some embodiments, the target-specific gRNA comprises multiple gRNA's. These multiple gRNA's may detect different parts of a target sequence, or may detect different target sequences. For example having multiple gRNA may be used to design a detection set that can detect multiple pathogens or multiple parts of pathogen.

The signal amplification of this embodiment comprises a POISER-oligo-specific guide RNA. The POISER-oligo-specific guide RNA can target a nucleic acid molecule of the nucleic acid molecule used in the amplification system, *e.g.* the POISER-oligo, preferably the POISER-oligo further comprising a polynucleotide tail. As a consequent, the POISER-oligo can be cleaved by the effector protein of the signal amplification system and/or of the CRISPR-Cas system.

The term "target-specific" and "POISER-oligo-specific" are used to distinguish the guide RNA of the CRISPR-Cas system used to recognise the target nucleic acid (target-specific guide RNA) and the guide RNA used in the signal amplification system (POISER-oligo-specific guide RNA).

In some embodiments the POISER-oligo-specific guide RNA is at least partially complementary to the polynucleotide tail. In some embodiments, the POISER-oligo-specific guide RNA is at least partially complementary to the polynucleotide tail and at least partially complementary to the POISER-oligo, thus at least partially complementary to the hybrid molecule. The hybrid molecule comprises the effector protein cleaved POISER-oligo with the polynucleotide tail.

The composition of the polynucleotide tail is determined by the nucleotides and the specific polymerase as provided in the system. If a single type of nucleotides is provided in the system, the polynucleotide tail will essentially comprise the provided nucleotides. The most common nucleotides are thymine, adenine, guanine, cytosine and/or uracil. For example, if the detection system only comprises thymine, the polynucleotide tail will essentially comprise thymine nucleotides.

The POISER-oligo-specific guide RNA is designed to be complementary to the polynucleotide tail. Thus, the type of nucleotides provided in the system must correspond to the design of the POISER-oligo-specific guide RNA. Thus, if the POISER-oligo-specific guide RNA comprises adenine residues as complementary strand the provided nucleotides in the system needs to be thymine and/or uracil. If the POISER-oligo-specific guide RNA comprises thymine and/or uracil residues as complementary strand the provided nucleotides in the system needs to be adenine. If the POISER-oligo-specific guide RNA comprises guanine residues as complementary strand the provided nucleotides in the system needs to be cytosine. If the POISER-oligo-specific guide RNA comprises cytosine residues as complementary strand the provided nucleotides in the system needs to be guanine.

The nucleic acid detection system of the invention comprises a template-independent polymerase. The polymerase exhibits catalytic activity capable of transferring nucleotides to termini of nucleic acid fragments, such as 3' hydroxyl termini, to form a polynucleotide tail. The polymerase is an enzyme which is capable of synthesising nucleic acid chains.

In embodiments, the polymerase is a template-independent polymerase, preferably DNA polymerase and/or RNA polymerase, more preferably terminal deoxynucleotidyl transferase (TdT) and/or Pοlθ terminal transferase or a derivative thereof.

The nucleic acid detection system of the invention comprises a polymerase. The polymerase exhibits catalytic activity capable of transferring nucleotides to termini of nucleic acid fragments, such as 3' hydroxyl termini, to form a polynucleotide tail. The polymerase is an enzyme which is capable of synthesising nucleic acid chains.

In embodiments, the polymerase is a template-independent polymerase. The template-independent polymerase refers to a polymerase that does not require a template to incorporate nucleotides. This type of polymerases are also called non-templated directed polymerases.

In preferred embodiments, the polymerase may comprise DNA polymerase and/or RNA polymerase, depending on the target nucleic acids, CRISPR-Cas system and/or nucleotide. For example, the polymerase may be terminal deoxynucleotidyl transferase (TdT) or Pοlθ terminal transferase.

Preferably, the polymerase comprises TdT and/or Pοlθ terminal transferase or a derivative thereof. TdT possesses the ability to incorporate nucleotides, in particular thymine, in a template-independent manner using single-stranded DNA or double-stranded DNA as the nucleic acid substrate. Preferably, single stranded DNA is used as it functions more efficiently as a template.

Template-independent DNA-synthesis/activity has also been reported for Pοlθ terminal transferase in addition to its template-dependent activity. Template-independent activity of Pοlθ terminal transferase requires manganese as a co-factor and is most efficient on DNA containing 3'overhangs.

In some embodiments, the nucleic acid detection system of the invention further comprises nucleotides and/or derivates thereof. Nucleotides, or nucleoside phosphates, consist of a nucleoside, which consists of a nucleobase, or nitrogenous base, and ribose, and one or more phosphate groups. As used herein, a nucleotide is a molecule which comprises a nitrogenous base and one or more phosphate groups bound to a ribose or deoxyribose. In particular, the nitrogenous base is pyrimidine or a derivative thereof. The nitrogenous base may be selected from thymine, cytosine, uracil, alloxan, inosine, adenine or derivatives thereof. For example, the nucleotide may be a labelled nucleotide that is conjugated to one or more moieties, such as fluorophores or biotin, that can be incorporated into DNA and RNA to facilitate detection or hybridisation. Preferably, the nucleotides comprise thymine. The nucleotides comprise at least one phosphate group. In particular, the nucleotides comprise 2 or more phosphate groups, such as 3 phosphate groups. Preferably, the nucleotides are nucleoside triphosphates. More preferably, the nucleotides are deoxythymidine triphosphates (dTTP) or derivatives thereof.

The polymerase and the nucleotide are selected as such that the polymerase is capable of polymerising the nucleotide to form a polynucleotide (tail).

In some embodiments the nucleic acid detection system or signal amplification system as described herein, further comprises nucleotides that correspond to the complementary sequence of the POISER-oligo-specific guide RNA.

According to the disclosure, a polynucleotide tail is formed and/or attached to the cleaved POISER oligo, in particular via the 3'-hydroxyl termini of the cleaved POISER oligo. As such, a polynucleotide attached to (cleaved) fragments is coined a polynucleotide tail. The polynucleotide tail as described herein may comprise at least 5 nucleotides, as described herein. The polynucleotide tail may comprise 100 nucleotides or more, such as 150 or more, or 200 or more. For example, the polynucleotide tail may comprise 100-500 nucleotides, such as 150-400, or 200-300. The polynucleotide tail may comprise 5 nucleotides or more and 100 nucleotides or less, such as 15-75, 20-70, or 25-65. Typically, a tail of at least 7 nucleotides is required for the guide RNA to recognise and/or hybridiSe. The polynucleotide tail may comprise different nucleotides, such as 2 or more different nucleotides, *e*.*g*., 3, 4 or 5 different nucleotides. The polynucleotide tail may comprise nucleotides thymine, adenine, guanine, cytosine and/or uracil. Preferably, the polynucleotide tail has only one type of nucleotide. For example, the polynucleotide tail is a thymine polynucleotide tail.

The term "target-activated nucleic acid cleavage activity" as used herein refers to activation of the effector protein by recognition of a target nucleic acid. Recognition is mediated by a guide RNA that binds, hybridises and/or pairs with a nucleic acid molecule of a target. The guide RNA in complex with the effector protein activates the cleavage activity of the effector protein. Thus the effector protein is target-activated and activates the cleavage activity.

The effector protein can cleave the target molecule. In addition, some effector proteins are capable of collateral cleavage. With this activity the effector protein can cleave other nucleic acids which are not the target molecule. For example this collateral cleavage activity can be used to cleave reporter molecules to generate nucleic acid fragments.

The effector protein exhibits target-activated nucleic acid cleavage activity. This activity results in cleavage of the target nucleic acid, *e.g.* a pathogenic nucleic acid. Besides cleavage of the target, the effector protein can also cleave other nucleic acid sequences, such as a nucleic acid reporter molecule or a POISER-oligo. This is called collateral cleavage activity.

Collateral cleavage activity, also referred to herein as "trans-cleavage activity", refers to a non-specific or sequence-independent cleavage activity. Collateral cleavage activity is an alternative activity of several Cas proteins. Some examples of Cas proteins with collateral cleavage activity are Cas12a, Cas13a, Cas14. Upon recognition of the target nucleic acid, the collateral cleavage of the effector protein is activated, so that the effector protein promiscuously/non-specifically cleaves non-target or unrelated nucleic acid. Depending on the type different Cas enzymes can collaterally cleave DNA, RNA or both. For example, Cas12a cleaves ssDNA and Cas13a cleaves RNA.

In the present disclosure collateral cleavage activity induces cleavage of the POISER-oligo. Collateral cleavage of the POISER-oligo is the start of the positive-feedback loop. Activation of the positive-feedback loop enhances the signal.

The nucleic acid detection system or signal amplification system of the invention preferably comprises reporter molecules. The reporter molecules can be cleaved, for example by a CRISPR-Cas enzyme. Cleavage results in a signal which is measurable, detectable or visible. The reporter molecules may be FRET (fluorescence resonance energy transfer, also known as Förster resonance energy transfer) reporter molecules. Upon cleavage these reporter molecules will produce a FRET signal. Detection and quantitation of FRET can certainly be accomplished in a number of different ways, for example with a spectrofluorometer.

The disclosure provides a nucleic acid detection system comprising a CRISPR-Cas system and a signal-amplification system. The signal amplification system can amplify the signal of the detection system. Using the amplification system allows detection of the target nucleic acid with a lower detection-limit.

For example, the known Cas12 diagnostics are reported to have a detection limit in the nanomolar to picomolar range. Using target-pre-amplification strategies the sensitivity of the lower detection-limit of these tests can reach femtomolar to even attomolar sensitivity. The present disclosure aims to increase the sensitivity of a nucleic acid detection system, without using pre-amplification strategies, but instead using a signal amplification system. In some embodiments the sensitivity of the nucleic acid detection system is increased to a femtomolar range, more preferably to an attomolar range, more preferably to a zeptomolar range.

In the present disclosure the signal is amplified using a positive feed-back loop. The term positive feed-back loop as used herein refers to a system that upon activation results in an increased signal. The positive feedback loop increases the number of active reporter molecules that are generated by the CRISPR-Cas system. As a result a lower number of target nucleic acids can result in a detectable or visible signal. Thus, the detection limit for the target nucleic acid is lower due to the positive feedback loop.

The positive feedback loop is based on a POISER-oligo. POISER stands for Partial Or Incomplete Sites for crRNA recognition. POISER-oligo's are oligonucleotides, which consist of the nucleotides such as thymine, adenine, guanine, cytosine and/or uracil. The POISER-oligo can also be called a POISER-oligo, or POISER-oligonucleotide. Preferably the POISER-oligo's are single stranded polynucleotides.

In some embodiments the POISER-oligo is single stranded DNA, single stranded RNA, or single stranded hybrid DNA-RNA, preferably single-stranded DNA.

In some embodiments the POISER-oligo's are blocked at the 3' terminus of the polynucleotide. This block prevents that POISER-oligo from being recognised by a polymerase and thus prevents elongation. Blocking at the 3' terminus can be any accomplished in several ways as long as it prevents it from being recognised by the polymerase. For example the block can be missing of a functional 3'OH, 3'ddC, 3' Inverted dT, 3'C3spacer, 3'Amino, circularisation and 3' phosphorylation. In some embodiments the POISER-oligo is blocked with synthetic 3' phosphorylation plus enzymatic addition of ddCTP [ddNTPs] plus TdT for complete block. In some embodiments, the POISER-oligo is blocked by circularisation.

The POISER-oligo's can be cleaved, preferably by the CRISPR-Cas system as described herein, in particular by the effector protein of the CRISPR-Cas system as described herein. Cleavage of the POISER-oligo results in a free 3'OH group at the 3' terminus of the polynucleotide. This 3' OH group can be recognised by a polymerase. The polymerase exhibits catalytic activity capable of transferring nucleotides to the cleaved POISER-oligo to form polynucleotide tails.

Four schematic examples of POISER-oligo's are shown in Fig. 1. These examples support the disclosure, but do not limit the scope of the invention.

POISER1 as displayed in Fig. 1 is a linear polynucleotide comprising nucleotides and a block on the 3' terminus.

POISER2 is a is a linear polynucleotide comprising nucleotides and a block on the 3' terminus, further comprising nucleotides at least partially complementary to the POISER-oligo-specific gRNA.

POISER3 is a circular molecule, also called a dumbbell. As the molecule is circular there is no open 3' terminus, thus blocking the 3' terminus. Cleavage of the circular polynucleotide will result in an open 3' terminus, which can be recognised by the polymerase. In some embodiments, the circular POISER-oligo comprises nucleotides at least partially complementary to the POISER-oligo-specific guide RNA.

POISER4 is a linear polynucleotide comprising nucleotides and a block on the 3' terminus, further comprising nucleotides at least partially complementary to the POISER-oligo-specific guide RNA. Furthermore, this POISER-oligo is a hybrid of DNA and RNA.

In some embodiments, the POISER-oligo comprises 4-20 nucleotides at least partially complementary to the POISER-oligo-specific guide RNA and wherein the POISER-oligo is blocked at the 3'-terminus.

In some embodiments, the POISER-oligo comprises 4-15 nucleotides at least partially complementary to the POISER-oligo-specific guide RNA and wherein the POISER-oligo is blocked at the 3'-terminus.

In some embodiments, the POISER-oligo comprises 9-15 nucleotides at least partially complementary to the POISER-oligo-specific guide RNA and wherein the POISER-oligo is blocked at the 3'-terminus.

In some embodiments, the POISER-oligo comprises 11-15 nucleotides at least partially complementary to the POISER-oligo-specific guide RNA and wherein the POISER-oligo is blocked at the 3'-terminus.

In some embodiments, the POISER-oligo comprises 13-15 nucleotides at least partially complementary to the POISER-oligo-specific guide RNA and wherein the POISER-oligo is blocked at the 3'-terminus.

In some embodiments, the POISER-oligo comprises 13 nucleotides of either G, C, A or T. The POISER sequence is irrelevant as long as the sequence is at least partially complementary to the POISER-oligo-specific guide RNA. Preferably, the POISER-oligo is up to 15 nucleotides. Longer POISER oligonucleotides may activate Cas-POISER complexes without the occurrence of pathogen recognition.

In some embodiments the POISER-oligo used is single-stranded DNA. In some embodiment the POISER-oligo used is single-stranded-RNA or hybrid DNA-RNA.

In some embodiments, the amplification system as disclosed herein, or the nucleic acid detection system as disclosed herein can be used in a fast point-of-care (POC) diagnostic DNA/RNA detection test. These test or diagnostics assays are developed for the detection of pathogens, oncogenes and/or genetic pathologies near or even at the point of care of the subject, for example at the bedside. Some examples are SHERLOCK variations (such as miSHERLOCK - minimally instrumented SHERLOCK), POC-CRISPR (SARS-CoV-2 detection test) and CANTRIP (a CRISPR-Cas FRET-based detection system that generates copper nanoparticles).

The POC testing is accomplished by integrating assays in (trans)portable devices. Ideally, the steps one needs to perform from raw sample to understandable result are minimised, allowing unskilled operator to perform analysis. In contrast to the conventional testing/detection tests in medical laboratory, the POC testing comprises of total-analysis device, integrating sample pre-treatment, target recognition/detection and signal acquisition in a single device. Thus, POC testing provides rapid detection, faster turn-around-times and avoidance of sample transport problems. Other advantages of POC testing include ease of handling, providing more efficient and faster diagnosis.

In some embodiments the POISER-oligo is at least partially protected with a backbone modification from degradation, preferably with a phosphorothioate (PS) modification or any other method for protection known to those of ordinary skill in the art.

The term "backbone modification" refers to a modification of an oligonucleotide to enhance nuclease resistance. In particular, the backbone modification protects the part of the POISER-oligo which is complementary to the corresponding guide RNA (POISERcrRNA complementary-sequence) from getting degraded by CRISPR-Cas cleavage activity. For example Cas12a trans-cleavage activity can degrade an unprotected POISER-oligo. A modified oligonucleotide has a modified and stabilised internucleotide linkage that is relatively resistant to nuclease degradation compared to a phosphodiester internucleotide linkage. The modified oligonucleotide may contain stabilised linkages, for example, phosphorothioate (PS), phosphorodithioate, methylphosphonate, methylphosphorothioate, phosphonoacetate, methylene, amide, morpholino or other known types of stabilised linkages. The modified oligonucleotide may have one or more stabilised linkages described herein.

In some embodiments, at least one or more nucleotides, preferably at the 3'-end, of the modified/POISER oligonucleotide are standard phosphodiester (PD) nucleotides. Due to the phosphodiester linkage, the PD nucleotide(s) at the 3'-terminus allow Cas, such as, cleavage. Preferably, the POISER-oligonucleotide contains two PD nucleotides at the 3'-end.

In preferred embodiments, the backbone modification is a PS modification. The term "PS modification" refers to a backbone modification in which a stabilised linkage is a phosphorothioate linkage. The phosphorothioate is a linkage in which a sulfur atom replaces a non-bridging oxygen atom in a phosphodiester linkage. Each phosphorothioate linkage can occur as either an Rp or Sp diastereomer. Such PS modification renders the internucleotide linkage resistant to nuclease degradation and enhances the stability of the oligonucleotides.

In some embodiments, the detection system is a nucleic acid fluorescence detection system, *e.g.* a FRET detection system. In some embodiments, the POISER-oligo is a nucleic acid fluorescence detection system or a FRET detection system.

The nucleic acid fluorescence detection system can be FRET detection system. The term "FRET" (fluorescence resonance energy transfer, also known as Förster resonance energy transfer) refers to a distance-dependent physical process by which energy is transferred non-radiatively from an excited donor fluorophore to an acceptor fluorophore by means of intermolecular long-range dipole-dipole coupling. The range over which the energy transfer can take place is limited to approximately 10 nanometers (10 nm or less). Further, the donor emission spectrum ideally overlaps the acceptor absorption spectrum, but there should be no overlap in the excitation spectra. Excitation of the donor therefore causes excitation of and emission from the acceptor. A pair of molecules that interact in such a manner that FRET occurs is often referred to as a donor/acceptor pair. Thus, a FRET signal serves as a proximity gauge of the donor and acceptor; only when they are in close proximity to one another a signal is generated.

In preferred embodiments the POISER-oligo is blocked at the 3'-terminus with a fluorophore or quencher, such as a fluorophore or quencher of a FRET reporter assay. Hence, in some embodiments, the POISER-oligo is a nucleic acid fluorescence detection system, such as a FRET detection system. Addition of a 3'-terminus block can also be effectively achieved using a fluorophore or quencher identical to those used in the FRET reporter. Therefore it seems credible that the FRET reporter itself can be constructed as a POISER-oligo. Thus after cleavage of this FRET reporter, the 5' part can function as a cleaved POISER from which the polymerase, such as TdT, starts polymerisation of a polynucleotide tails, such as a T-tail (Fig. 3). Thus the reporter molecule itself can also function as the POISER-oligo.

In preferred embodiments, the CRISPR-Cas system comprises a Cas enzyme with collateral cleavage activity (Cas12, Cas13, Cas14) as effector protein, preferably wherein the effector protein is Cas12, more preferably wherein the effector protein is Cas12a.

CRISPR-Cas systems are commonly categorised in two classes (1 and 2). Types and subsequent subtypes constitute either class. The categorisation of CRISPR-Cas systems is based on, for example, the constitution of effector modules of single, large, multidomain proteins that are generally derived from genetic elements. Whereas class 1 CRISPR-Cas systems contain a multi subunit Cas nuclease, or effector protein, class 2 CRISPR-Cas systems are characterised by a single subunit effector protein. Exemplary effector proteins that belong to class 2 CRISPR-Cas systems are type II Cas9 and Cas9-like proteins , type V Cas12 and Cas12-like proteins, such as subtype V-A Cas12 (Cpf1, or Cas12a), subtype V-B Cas12 (Cas12b, or C2c1), subtype V-C Cas12 (C2c3) and subtype V-F Cas12 (Cas12f, or Cas14 or C2c10) and type VI Cas13 and Cas13-like proteins, such as Cas 13a (C2c2) and Cas13b (C2c6).

In embodiments, the CRISPR-Cas system comprises a Cas enzyme with collateral cleavage activity as effector protein. Collateral cleavage activity, also referred to herein as "trans-cleavage activity", refers to an alternative activity of several Cas proteins. Upon recognition of the target sequence, the Cas protein's collateral activity is activated, so that the Cas enzyme promiscuously/non-specifically cleaves non-target or unrelated nucleic acid (DNA or RNA or both, depending on the Cas enzyme). Cas enzymes with collateral cleavage activity can be selected from class 1 type III (such as Cas10, Csm6), class 2 type V (such as Cas12 and Cas12-like proteins) or class 2 type VI (such as Cas13 and Cas13-like proteins). Preferably, the CRISPR-Cas system is a type V or VI system. More preferably, the CRISPR-Cas system is a class 2 type V system. In case of a class 2 system, the effector protein may particularly be Cas12 or an effector protein having similar cleavage activity as Cas12. The Cas12 or Cas12-like effector protein may, for example, be selected from Cas12a, Cas12b, Cas12c, Cas12d, Cas12e and Cas12f. Preferably, the CRISPR-Cas system comprises a C12a effector protein or an effector protein having similar cleavage activity as Cas12a.

In some embodiments, the target sequence of the target comprises nucleic acids.

The target sequence may be located in the nucleus, organelles or cytoplasm of a cell, and may include nucleic acids in or from mitochondrial, organelles, vesicles, liposomes or particles present within the cell. The target sequence does not have to be located intracellular. For example, the target sequence may be extracellular, such as in spores. In case the target sequence is a virus sequence, it may be located in a protein structure, such as a viral capsid.

In preferred embodiments the target sequence may be any DNA sequence or RNA sequence. The target sequence may be a sequence within RNA selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (IncRNA), and small cytoplasmic RNA (scRNA).

The target sequence may be any DNA sequence. The target nucleic acids may then comprise, or consist essentially of, DNA.

Preferably, the target sequence is single-stranded DNA (ssDNA) or double-stranded DNA (dsDNA). In particular, in the case of Cas12a nuclease, the target sequence may be any DNA sequence, for example, any sequence of ssDNA or dsDNA.

In case of ssDNA, there may not be any preference or requirement for a protospacer adjacent motif (PAM) sequence in the target DNA. However, when the target DNA is dsDNA, a PAM is usually present adjacent to the target sequence of the target DNA. The source of the target DNA can be any source as described herein, such as obtained from any sample as described herein.

In some embodiments the target sequence of the target comprises nucleic acids of a pathogen.

In some embodiments the target sequence of the target comprises nucleic acids of an oncogene and/or genetic pathology.

The target sequence can be a proto-oncogene or an oncogene.

The target sequence can include other genetic abnormalities /mutations involved in genetic pathology. Thus, such target sequence can comprise nucleic acids indicative of/diagnostic for a particular physiological state, disease state and/or condition (e.g., presence or state of a disease, disorder or condition such as, for example, an inflammatory or metabolic disease, disorder or condition, *etc*.). In particular, the disease state is selected from infectious diseases, organ diseases, blood diseases, immune system diseases, cancers, brain and nervous system diseases, endocrine diseases, pregnancy or childbirth-related diseases, inherited diseases, environmentally-acquired diseases, or a combination thereof, preferably infectious diseases.

Preferably, the target sequence comprises nucleic acids of a pathogen. Examples of nucleic acids from a pathogen or an infectious agent causing the infectious disease are, for example, viral DNA, viral RNA, parasite DNA, bacterial DNA.

The target DNA can be a viral DNA (*e*.*g*., a genomic DNA of a DNA virus). As such, the system, agents, devices, kits and methods as described herein can each be used for detecting the presence of a viral DNA amongst a population of nucleic acids in any sample, as described herein.

Examples of target DNAs include viral DNAs selected from papovavirus, such as human papillomavirus (HPV) and polyoma virus; hepadnavirus, such as Hepatitis B Virus (HBV); herpesvirus, such as herpes simplex virus (HSV), varicella zoster virus (VZV), epstein-barr virus (EBV), cytomegalovirus (CMV), herpes lymphotropic virus, Pityriasis Rosea, and kaposi's sarcoma-associated herpesvirus; adenovirus, such as atadenovirus, aviadenovirus, ichtadenovirus, mastadenovirus, and siadeno virus; poxvirus, such as smallpox, vaccinia virus, cowpox virus, monkeypox virus, orf virus, pseudocowpox, bovine papular stomatitis virus, tanapox virus, yaba monkey tumour virus, molluscum contagiosum virus (MCV); parvovirus, such as adeno-associated virus (AAV), Parvovirus B19, human bocavirus, bufavirus, and human parv4 Gl); Gemini viridae; Nanoviridae; and Phycodnaviridae.

The target DNA may be parasite DNA. The target DNA may be bacterial DNA, *e*.*g*., DNA of a pathogenic bacterium, such as DNA of Bacillus anthracis. The target DNA may be from any gram-negative bacterium or gram positive bacterium, such as a *Mycobacterium.* As such, the system, agents, devices, kits and methods as described herein can each be used for detecting the presence of a bacterial DNA amongst a population of nucleic acids in any sample, as described herein.

Examples of target DNAs include bacterial DNAs selected from the bacteria genera *Streptococcus; Staphylococcus; Pseudomonas; Chlamydophila; Ehrlichia; Rickettsia; Orientia; Yersinia; Burkholderia; Shigella; Campylobacter; Salmonella; Clostridium; Corynebacterium; Treponema; Neisseria; Brucella; Mycobacterium; Lactobacillus; Nocardia; Listeria; Francisella,* and *Legionella.*

The invention further provides use of the nucleic acid detection system as described herein as a diagnostic test. The diagnostic test may be used to diagnose a pathological state, disease state and/or condition of a subject as described herein.

The invention further provided use of the signal amplification system as described herein in a diagnostic test. Preferably the use of the signal amplification system and a nucleic acid detection system as described herein as a diagnostic test.

The invention further provides a method for detecting and/or monitoring nucleic acids in a subject, particularly *ex vivo* or *in vitro,* wherein preferably the nucleic acids are microbial nucleic acids, more preferably pathogenic nucleic acids. The method comprises the steps of the method for detecting (target) nucleic acids as described herein. With this method, a sample, such as a biological sample as described herein, is taken from a subject for detecting and/or monitoring nucleic acids that are present in the sample. Preferably, microbial nucleic acids, and more preferably pathogenic nucleic acids, are detected and/or monitored. Even more preferably, the method is used for detecting and/or monitoring (the presence of) pathogenic DNA, such as viral and/or bacterial DNA, in a subject.

The invention further provides a method for diagnosing a disease state of a subject, particularly *ex vivo* or *in vitro.* The method comprises the steps of the method for detecting nucleic acids as described herein. The disease state is preferably selected from the disease states as described herein.

The present disclosure further provides a method for detecting one or more a nucleic acids of interest in a sample, wherein methods comprises a CRISPR-Cas system and a signal amplification system. This method has a high sensitivity to detect a target sequence of a target, but omitting extra pre-amplification and waiting steps. In particular, the method has a low detection limit for a nucleic acid target, in particular nucleic acids of pathogens, oncogenes and/or genetic pathologies.

The disclosure further provides a method for detecting one or more nucleic acids of interest in a sample, wherein the method comprises a CRISPR-Cas system and a signal amplification system, the method comprising the following steps:
i) adding the CRISPR-Cas system, signal amplification system,
   template-independent-polymerase, and a reporter molecule as described herein to a sample,
ii) read-out of the reporter.

In preferred embodiments, the method comprises adding nucleic acids to the sample, preferably nucleic acids corresponding with the polymerase.

Adding the CRISPR-Cas system, signal amplification system, template-independent-polymerase, and a reporter molecule as described herein to a sample will initiate a reaction. If the sample comprises the nucleic acids of interest the reaction will result in a signal.

Read-out of the reporter can be performed by any method as known to the skilled person. Some read-out methods are used after the reaction has taken place, for example Lateral flow assays (LFA), *e.g.* LFA-strip. Alternatively, some read-out methods can be performed during the reaction. For example measuring a FRET signal. In preferred embodiments, the read-out of the reporter takes place during the reaction.

The disclosure further provides a method for detecting one or more nucleic acids of interest in a sample, wherein methods comprises a CRISPR-Cas system and a signal amplification system, the method comprising the following steps:
i) activation of nucleic acid cleavage activity of the CRISPR-Cas system by a target sequence of a target, resulting in cleavage of the target sequence and collateral cleavage activation,
ii) collateral cleavage of a POISER-oligo blocked at the 3'-terminus resulting in removal of the 3' block,
iii) transfer of nucleotides to the cleaved POISER-oligo forming a polynucleotide tail at the 3' terminus of the POISER-oligo by polymerase exhibiting catalytic activity, resulting in a POISER-polynucleotide-hybrid;
iv) recognition of the POISER-polynucleotide-hybrid by a POISER-oligo-specific guide RNA having a guide sequence being capable of targeting the CRISPR-Cas system to the POISER-polynucleotide-hybrid;
v) activation of the nucleic acid cleavage activity of the CRISPR-Cas system, resulting in cleavage of the POISER-nucleotide hybrid and collateral cleavage of a reporter molecule to generate a signal.

The CRISPR-Cas system and the signal amplification system may be as described in the embodiments of the present application. For example the CRISPR-Cas system comprises an effector protein and a target-specific guide RNA having a guide sequence being capable of targeting the effector protein to a target sequence of a target, and the effector protein exhibiting target-activated nucleic acid cleavage activity capable of cleaving nucleic acid reporter molecules to generate nucleic acid fragments.

For example the signal amplification system comprises a partial crRNA recognition site-oligo (POISER-oligo) and a POISER-oligo-specific guide RNA having a guide sequence being capable of targeting the effector protein to an POISER-oligo with polynucleotide tail, and the effector protein exhibiting target-activated nucleic acid cleavage activity capable of cleaving nucleic acid reporter molecules to generate nucleic acid fragments.

Furthermore the method uses a polymerase exhibiting catalytic activity. This polymerase may be as described as in de embodiments of the present application.

Target-activation, or effector protein activation, is achieved by targeting (guiding) the effector protein of the CRISPR-Cas system to a specific target sequence, wherein the target may be a target nucleic acid as described herein. Preferably, the target is a pathogenic nucleic acid. Target-activation may comprise providing the CRISPR-Cas system to a sample, such as any sample described herein. If the sample contains the target sequence, then the CRISPR-Cas system will be activated and will subsequently cleave the nucleic acid reporter molecules.

In particular, the nucleic acid reporter molecule in the method is as described herein. The nucleic acid reporter molecule is preferably blocked at the 3' terminus to prevent, for example, elongation of the polymerase in the absence of nuclease activity. Preferably, when the CRISPR-Cas system comprises Cas12a as the effector protein, the nucleic acid reporter molecule is ssDNA, in particular ssDNA having their 3'-termini blocked. The low cost and adaptability of the detection system as described herein lends itself to a number of applications. The generated fragments preferably have 3'-hydroxyl ends such that polynucleotide tails are attached at said ends.

With step i) of the method the temperature at which the target activation and/or generation of fragments is performed depends on the effector protein. For example, the step may be performed at room temperature. In particular, the step may be performed at a temperature of at least 20 °C, such as 25-60 °C. Preferably, step i) is performed at a temperature of 30-55 °C to assist the formation of nucleic acid reporter fragments. The CRISPR-Cas system may be target-activated during at least 1 min. In particular, the duration of step i) may be 2 min or more, preferably at least 15 min. In some embodiments, the duration of step i) may be 90 min or less, such as 60 min or less, 40 min or less, 35 min or less, 30 min or less, 25 min or less, 20 min or less, or 15 min or less, for example, 3-50 min, or 4-45 min. Preferably, the duration of step i) is approximately 5-40 min, such as 6-35 min or 7-30 min. Shortening the duration of 60 min of step i) allows for faster total detection, yet results in a decreased signal. In a preferred embodiment, the duration of step i) is between 40-70 min.

Step i) of the method as described herein may be performed in weak basic conditions, i.e., at a pH of 7.5 or higher. In particular, the pH is 7.6 or higher and 9 or lower. Preferably, the pH is about 7.7-8.5. A buffer may be present at any step of the method as described herein. For example, the buffer may contain acetate, having a pH of 7-9, such as about 7.5-8.5. The buffer may comprise a cation, such as divalent metal ions, *e*.*g*., Mg²⁺, as it may be required by the polymerase and/or effector protein. Other divalent metal ions that may be present in the buffer are Co²⁺, Mn²⁺ and/or Zn²⁺. In an exemplary embodiment using Cas12a as the effector protein and TdT as the polymerase, an acetate containing buffer is used.

In some embodiments the POISER-oligo is blocked at the 3'-terminus with a fluorophore or quencher of a FRET reporter assay. Addition of a 3'-terminus block can also be effectively achieved using a fluorophore or quencher identical to those used in the FRET reporter. Therefore it seems credible that the FRET reporter itself can be constructed as a POISER-oligo. Thus after cleavage of this FRET reporter, the 5' part can function as a cleaved POISER from which TdT starts polymerisation of a T-tail (Fig. 3). Thus the reporter molecule itself can also function as the POISER-oligo.

In some embodiments, the sample is a biological sample and/or an environmental sample. The biological sample or environmental sample may originate from a subject as described herein. The biological sample may be obtained from blood, plasma, serum, urine, stool, sputum, mucous, lymph fluid, synovial fluid, bile, ascites, pleural effusion, seroma, saliva, cerebrospinal fluid, aqueous or vitreous humour, or any bodily secretion, a transudate, an exudate, such as fluid obtained from an abscess or any other site of infection or inflammation), or fluid obtained from a joint, such as a normal joint or a joint effected by disease, such as rheumatoid arthritis, osteoarthritis, gout or septic arthritis, a swab of skin or mucosal membrane surface, or a combination thereof. Preferably, the biological sample may be obtained from blood, plasma, serum, urine, stool, sputum, mucous, saliva, or any bodily secretion, a transudate, an exudate, such as fluid obtained from an abscess or any other site of infection or inflammation), a swab of skin or mucosal membrane surface, or a combination thereof. The environmental sample may be obtained from food (*e*.*g*., fruit, vegetables, meat, beverage, *etc*.), paper surface, fabric, metal surface, wood or wood surface, plastic surface, soil, water, such as fresh water or waste water, saline water, atmospheric air or other gas sample, or a combination thereof.

The disclosure further provides a kit of parts comprising the CRISPR-Cas system, the signal amplification system and the template-independent polymerase as disclosed herein.

The disclosure further provides a kit of parts comprising the amplification system and the template-independent polymerase as disclosed herein.

In some embodiments the kit of parts may further comprises nucleotides, instructions, containers containing the components of the CRISPR-Cas system and/or the signal amplification system.

The term "guide sequence" as used herein is meant to refer to a nucleotide sequence of a guide RNA molecule. The guide sequence is considered to be the proverbial key to bind an effector protein, comprising the guide RNA, to a target molecule, *e*.*g*., a nucleic acid, through sequence pairing.

The term "nucleic acid" as used herein is meant to refer to a polymeric form of nucleotides of any length, *e*.*g*., ribonucleotides and deoxyribonucleotides. The term is used to include microbial nucleic acids, such as pathogenic nucleic acids. The term encompasses single-stranded DNA, double-stranded DNA, multi stranded DNA, single-stranded RNA, double-stranded RNA, multi-stranded RNA, genomic DNA, cDNA, DNA RNA hybrids, and polymers comprising purine and pyrimidine bases or other natural, chemically or biochemically modified, non-natural, or derivatised nucleotide bases.

The term "protein" as used herein is meant to refer to a polymeric form of amino acids of any length, which can include coded and non-coded amino acids, chemically or biochemically modified or derivatised amino acids, and proteins having modified backbones.

The term "target sequence" as used herein is meant to refer to a nucleotide sequence of a target (*i*.*e*., a target molecule) to which a guide RNA having a sequence with a certain degree of complementarity with the target sequence, may bind an effector protein to the target (molecule).

The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open ended terms (*i*.*e*., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. For the purpose of the description and of the appended claims, except where otherwise indicated, all numbers expressing amounts, quantities, percentages, and so forth, are to be understood as being modified in all instances by the term "about". Also, all ranges include any combination of the maximum and minimum points disclosed and include any intermediate ranges therein, which may or may not be specifically enumerated herein.

When referring to a noun (*e*.*g*., an effector protein) in the singular, the plural is meant to be included, or it follows from the context that it should refer to the singular only.

For the purpose of clarity and a concise description features are described herein as part of the same or separate embodiments, however, it will be appreciated that the scope of the invention may include embodiments having combinations of all or some of the features described.

The invention is further explained in the following examples. These examples do not limit the scope of the invention, but merely serve to clarify the invention.

### EXAMPLES

### Example 1

### POISER1

The target sequence of the POISER1crRNA (20A's) is present numerous times in the large TdT generated poly-T tails, and the POISER design is independent on the specific DNA sequence of the chosen DNA-POISER. Upon cleavage of the POISER and subsequent TdT elongation, Cas12a-POISER1crRNA binds to the poly-T tails activating trans-cleavage.

Considering the high efficiency of dNTPs incorporation by TdT *in vitro,* elongated POISER1 will have multiple sites that can activate Cas12a-POISER1crRNA. Hence, a single cleaved DNA-POISER1 oligo can activate many CRISPR-Cas complexes making this theoretically the most sensitive strategy of the two POISERs tested. This sensitivity, however, may make POISER1 sensitive to unwanted noise, since any ssDNA molecule with a free 3'OH end will be a potential activator for many Cas proteins after TdT elongation. Therefore, the ssDNA POISER1-oligos used in this assay for the readout and signal amplification must be completely blocked at the 3' end, since a small fraction of unblocked ssDNA oligos can potentially kick-start the positive feedback loop. When testing various blocked oligonucleotides, it was found that minute impurities in the form of non-blocked/inefficiently blocked ssDNA-oligos in the reaction forms a trigger for Cas12a-POISER1crRNA activation after TdT-dTTP elongation, even in the absence of target pathogen. POISER1 results in a very sensitive system, but a higher risk of falls positive test results.

### Example 2

The crRNA of POISER2, POISER2crRNA, is partially complementary to the POISER2-oligo and partially complementary to the added poly-T tail (Fig. 1&2). An advantage is that only cleaved and TdT-elongated POISER2-oligos are recognised by the POISER2crRNA, and subsequently activate Cas12 collateral DNase-activity. Therefore, the POISER2 system is more robust to ssDNA impurities than POISER1. However, with this approach one elongated POISER2 oligo forms only one new recognition site for the Cas12-POISER2crRNA complex, while for POISER1 a single DNA molecule with a poly-T tail could activate multiple Cas12a complexes (Fig. 1).

The Cas cleavage-site on the POISER2-oligo is preferably well defined, since the recognition site of POISER2crRNA requires a precise transition of the specific sequence on the POISER2-oligo followed by the poly-T tail. By protecting part of the POISER2-oligo from Cas-cleavage, the unprotected nucleotides will remain accessible and therefore cleavage can be directed at these specific position(s). In order to protect the POISER2crRNA complementary-sequence and to specifically direct Cas12 cleavage, part of the POISER2-oligo is protected using a phosphorothioate (PS) backbone, which is not cleavable by DNase activity.

Previous studies have shown that phosphorothioate (PS) bonds can inhibit Cas12a cleavage activity: extensions on the 3' or 5' end of crRNA inhibit Cas12a trans-cleavage activity, and non-specific phosphorothioate DNA oligos can inhibit Cas12a based genome editing. However, the effect of a PS-backbone in the recognition site of Cas12a-crRNA, is unknown.

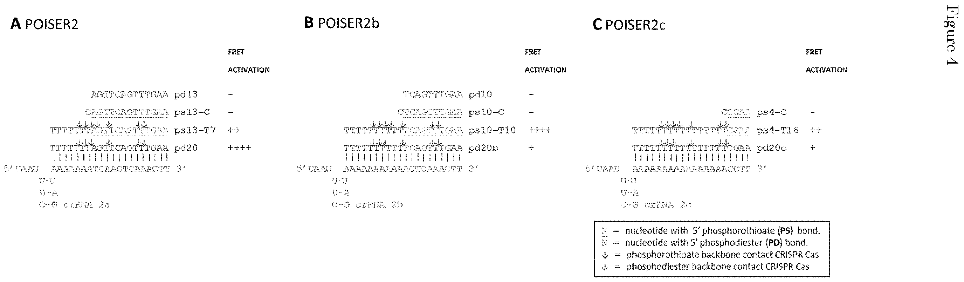

From the crystal-structure of the Cas12a, crRNA, and dsDNA interface, it is shown that Cas12a interacts with the DNA phosphodiester (PD) backbone of certain nucleotides (Fig. S1 of Swarts & Jinek (2019)). It is hypothesised that these PD-backbone interactions are needed for the proper formation of the crRNA-DNA heteroduplex and that a PS-backbone alteration in the crRNA target sequence could hinder the formation of these interactions, thereby inhibiting efficient Cas12a trans-cleavage activation. Therefore the effect of a PS-backbone on Cas12a-crRNA activation is determined. Three crRNA-ssDNA POISER-oligo pairs with decreasing numbers of PS-nucleotides were designed and tested in a Cas12a FRET reporter-assays (POISER2, 2b, 2c, Fig. 3.). Control reactions with PD-nucleotides targets are included. POISER2a contains 13 PS-nucleotides (orange underlined in Fig. 4) from which the backbones of 4 nucleotides are involved in the DNA-protein interactions (red arrows in Fig. 4). For POISER2b and POISER2c these numbers are 9 and 4 PS-nucleotides from which the backbones contacts of 2 and 0 nucleotides are involved in the DNA-protein interaction.

The measured FRET cleavage activity differed between PS-oligos and PD-oligos for all three POISER2 variants (Fig. 4). For POISER2, which has 4 protein-DNA backbone interactions involving PS-nucleotides (red arrows in Fig. 4), the activity is highest when the oligo contains no PS-backbone and consists entirely of a custom PD-backbone (PD-oligo). The activity is less when the PS-oligo contains 13 PS-groups in the backbone. This suggests that a PS-backbone in the target sequence negatively affects the complex formation and activation of the Cas12a-crRNA enzyme. In contrast, the activation measured with POISER2b and POISER2c is highest in the case of the PS-backbone compared to the corresponding PD-backbone oligo (with 2 and 0 predicted PS-backbone contacts respectively, red arrows in Fig. 4). Thus, lowering the number of PS-backbone groups which are involved in protein-DNA interactions, effectively reduces the negative effect on activity in POISER2b and POISER2c. Surprisingly, the measured activity of the PS-oligo's is even higher compared to the PD-oligo's for POISER2B. It is speculated that the partial PS-backbone protects the DNA from getting degraded by Cas12a trans-cleavage activity. As (incubation) time progresses, the concentration of the ssDNA oligo is, therefore, higher in the case of the protected PS-oligo than the PD-oligo.

### Example 3

To allow poly-T elongation of the POISER2-oligonucleotide by TdT, the 3' end-block should be removed by pathogen-activated Cas12a. Therefore, the last nucleotides need to have a PD-backbone to enable Cas12a cleavage. In a letter to the editor, Li *et al.* (2018) showed that the smallest 3' end fraction cleaved by active Cas12a is 2-nucleotide long. It is tested what would be the minimal required number of PD-nucleotides at the 3' end of the POISER2 oligonucleotide of which the remainder consisted of a PS-backbone (Fig. 5).

It was found that the signal produced with an oligo containing 2 PD-nucleotide overhang is much stronger than that of 1 PD-nucleotide overhang, which is in line with the observation by Li *et al.*

From these results, an optimal POISER2 oligonucleotide can be designed and built. The optimal design is POISER2b, which is short enough to omit the negative effect of backbone PS-groups on the protein-DNA interaction of Cas12a, while still ensuring the binding of the POISER2crRNA to the POISER2 oligonucleotide. The number of specific nucleotides in the POISER2 has to be short enough to not to activate the POISERcrRNA-Cas enzyme without TdT elongation. Moreover, the POISER2-oligonucleotide should contain 1, 2 or more PD-nucleotides at the 3' end to allow Cas12a cleavage. An additional benefit of having two PD-nucleotides at the 3' end is that these might prevent unwanted initiation of the POISER2 feedback loop caused by improperly blocked POISER2.

### Example 4

It is studied whether a POISER2b could increase the sensitivity of a Cas12a detection assay by adding, POISER2b oligonucleotide with two 3' terminal PD-nucleotides plus a POISER2b crRNA to a CRISPR-Cas12a reactions consisting of pathogen DNA (pALF), ALF crRNA, Cas12a and TdT and a blocked FAM-20C reporter (Fig. 6).

The detection limit of the POISER-free assay with a F-10C reporter is 10 pM (Fig. 6, without POISER2b) , while in the presence of POISER2b a signal is observed at 0.1 pM, showing that the feedback loop is active. These preliminary results show a proof-of-concept of a POISER feedback loop using POISER2b.

### Example 5

### POISER3

To allow poly-T elongation of the POISER-oligo by TdT, a new 3' OH end should be created by pathogen-activated Cas12a. Instead of chemically blocked oligo's, circular oligo's lacking free 3'OH ends can be used (*e*.*g*. POISER3, Fig. 1). Here a partial single stranded and partial double stranded oligonucleotide (dumbbell) is depicted. As CRISPR-Cas12 collateral activity targets mostly single stranded DNA, the site of Cas cleavage can be directed such that a gRNA can be designed at potential Cas cleavage sites where the poly-T tail will be added. Completely circular DNA POISER design should be possible but would require some backbone protection as described for POISER2.

### POISER4

The class 2 Type VI CRISPR-Cas13 prefers to cut single-stranded regions in RNA, rather than ssDNA. TdT in contrast does not use RNA as substrate. Therefore, in order to use Cas13 as well as TdT, a hybrid POISER DNA-RNA molecule should be used. (POISER4, Fig. 1). Allowing cleavage by Cas13 and elongation by TdT, is now largely identical to the POISER2 approach.

### References:

Notomi et al. Loop-mediated isothermal amplification of DNA. Nucleic Acids Res. 2000 Jun 15;28(12):E63.
Gootenberg et al. Nucleic acid detection with CRISPR-Cas13a/C2c2. Science. 2017 Apr 28;356(6336):438-442. doi: 10.1126/science.aam9321.
Shi et al. A CRISPR-Cas autocatalysis-driven feedback amplification network for supersensitive DNA diagnostics. Sci Adv. 2021 Jan 27;7(5):eabc7802. doi: 10.1126/sciadv.abc7802.

## Claims

1. A nucleic acid detection system, comprising a clustered regularly interspaced short palindromic repeat (CRISPR)- CRISPR-associated nuclease (Cas) system (CRISPR-Cas system), and a signal amplification system, wherein:
- the CRISPR-Cas system comprises an effector protein and a target-specific guide RNA (gRNA) comprising a guide sequence capable of targeting the effector protein to a target sequence, and the effector protein exhibiting target-activated collateral nucleic acid cleavage activity;
- the signal amplification system comprises a partial CRISPR RNA (crRNA) recognition site-oligo (POISER-oligo) and a POISER-oligo-specific gRNA comprising a guide sequence capable of targeting an effector protein to the POISER-oligo further comprising a polynucleotide tail, and the effector protein exhibiting target-activated collateral nucleic acid cleavage activity;
and a template-independent polymerase.

2. A signal amplification system for a nucleic acid detection system, comprising
- a partial crRNA recognition site-oligo (POISER-oligo),
- a POISER-oligo-specific guide RNA (gRNA) comprising a guide sequence capable of targeting an effector protein to the POISER-oligo further comprising a polynucleotide tail; and
- a non-template directed polymerase.

3. The nucleic acid detection system according to claim 1 or signal amplification system according to claim 2, wherein the POISER-oligo comprises 9-15 nucleotides at least partially complementary to the POISER-oligo-specific guide RNA.

4. The nucleic acid detection system or signal amplification system according to any one of the preceding claims, wherein the POISER-oligo is blocked at the 3'-terminus, *e*.*g*. by phosphorylation, circularisation, RNA nucleotides, 3'ddC, 3' Inverted dT, 3'C3spacer, 3'amino, and 3' phosphorylation.

5. The nucleic acid detection system or signal amplification system according to any one of the preceding claims, wherein the POISER-oligo is single stranded DNA, single stranded RNA, or single stranded hybrid DNA-RNA, preferably single-stranded DNA.

6. The nucleic acid detection system or signal amplification system according to any one of the preceding claims, wherein the POISER-oligo is at least partially protected with a backbone modification from degradation, preferably with a phosphorothioate backbone modification.

7. The nucleic acid detection system or signal amplification system according to any one of the preceding claims, further comprising a reporter molecule.

8. The nucleic acid detection system or signal amplification system according to any one of the preceding claims, wherein the POISER-oligo is a nucleic acid fluorescence detection system or a FRET detection system, preferably wherein the POISER-oligo is blocked at the 3'-terminus with a fluorophore or quencher of a FRET reporter assay.

9. The nucleic acid detection system according to any one of claims 1 or 3-7, wherein the CRISPR-Cas system comprises a Cas enzyme with collateral cleavage activity as effector protein, preferably wherein the effector protein is a class 2 type V or type VI Cas enzyme, preferably Cas12, Cas13, and/or Cas14, more preferably wherein the effector protein is Cas12a.

10. The nucleic acid detection system or signal amplification system according to any one of the preceding claims, wherein the effector protein of the CRISPR-Cas system and the effector protein of the signal amplification system are identical.

11. The nucleic acid detection system or signal amplification system according to any one of the preceding claims, wherein the template independent polymerase comprises a non-template directed DNA polymerase and/or a non-template directed RNA polymerase, more preferably terminal deoxynucleotidyl transferase (TdT) and/or Pοlθ terminal transferase or a derivative thereof, preferably the nucleic acid detection system or signal amplification system further comprises nucleotides.

12. The nucleic acid detection system according to any one of claims 1, 3-9, wherein the target sequence comprises nucleic acids, preferably DNA and/or RNA, preferably single stranded or double stranded DNA.

13. The nucleic acid detection system according to any one of claims 1, 3-10, wherein the target sequence comprises nucleic acids of a pathogen, oncogene and/or genetic pathology, preferably a nucleic acid of a pathogen.

14. Use of the nucleic acid detection system according to any of claims 1, 3-11 as a diagnostic test.

15. A method for detecting one or more nucleic acids of interest in a sample, wherein the method comprises a CRISPR-Cas system and a signal amplification system, the method comprising the following steps:
i) adding to a sample the CRISPR-Cas system, signal amplification system, template-independent-polymerase and a reporter molecule according to any one of the preceding claims,
ii) read-out of the reporter.
